(19) Europäisches Patentamt
European Patent Office
Office européen des brevets

(11) EP 2 999 690 B1

(12) **FASCICULE DE BREVET EUROPEEN**

(45) Date de publication et mention
de la délivrance du brevet:
**19.12.2018 Bulletin 2018/51**

(21) Numéro de dépôt: **14731695.4**

(22) Date de dépôt: **20.05.2014**

(51) Int Cl.:
**C07C 69/734** (2006.01)     **C09D 4/00** (2006.01)
**C09D 4/06** (2006.01)     **C08F 222/10** (2006.01)

(86) Numéro de dépôt international:
**PCT/FR2014/051174**

(87) Numéro de publication internationale:
**WO 2014/188117 (27.11.2014 Gazette 2014/48)**

(54) **PRODUITS ETHER-ESTERS ACRYLES MULTIFONCTIONNELS, PROCÉDÉ DE PRÉPARATION ET COMPOSITIONS RÉTICULABLES LIÉES**

MULTIFUNKTIONELLE ACRYLIERTE ETHER-ESTER-PRODUKTE, VERFAHREN ZUR HERSTELLUNG DAVON UND ZUGEHÖRIGE VERNETZBARE ZUSAMMENSETZUNGEN

MULTIFUNCTIONAL ACRYLATED ETHER-ESTER PRODUCTS, PROCESS FOR PREPARING SAME AND RELATED CROSSLINKABLE COMPOSITIONS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.05.2013 FR 1354651**

(43) Date de publication de la demande:
**30.03.2016 Bulletin 2016/13**

(73) Titulaire: **Arkema France**
**92700 Colombes (FR)**

(72) Inventeur: **CICERON, Philippe**
**F-60300 Senlis (FR)**

(74) Mandataire: **Killis, Andréas**
**ARKEMA France**
**Département Propriété Industrielle**
**420, rue d'Estienne d'Orves**
**92705 Colombes Cedex (FR)**

(56) Documents cités:
**JP-A- 2010 024 380     US-A- 5 243 069**

**Description**

La présente invention concerne des nouveaux monomères acrylés multifonctionnels qui sont des produits éthers-esters acrylés à base d'un mélange de produits acrylés multifonctionnels dérivés de la réaction de l'acide acrylique en défaut avec un polyol multifonctionnel, un procédé de préparation, des compositions réticulables à base de ces produits, des produits finis et utilisations desdits produits acrylés comme liants acrylés multifonctionnels de fonctionnalité élevée pour des compositions réticulables de haute densité de réticulation et à faible retrait et plus particulièrement pour des compositions de revêtements pigmentés ou non, en particulier des peintures, vernis, encres, adhésifs ou des compositions de moulage, d'étanchéité ou de composite ou de scellement chimique.

Des monomères acrylés multifonctionnels de fonctionnalité élevée, d'au moins 3 et pouvant aller jusqu'à 6, en groupements acrylates existent déjà et sont utilisés dans des applications de revêtements comme les vernis ou les encres pour augmenter la densité de réticulation et les performances liées, de résistance chimique ou de dureté.

JP 2010 024380 divulgue des compositions réticulables sous l'action de rayonnement ultra-violet et divulgue le produit résultant de l'addition de Michaël entre le pentaérythritol triacrylate et le pentaérythritol tetraacrylate.

Cependant, les monomères multifonctionnels acrylés existants conduisent à une mauvaise flexibilité, en particulier pour l'application revêtements, ladite flexibilité étant définie ici en termes de résistance à la pliure déterminée par le test à la pliure sur un support conique. Ainsi, le compromis dureté/flexibilité est mauvais ainsi que l'adhérence sur substrats, par exemple dans des applications pour revêtements tels que vernis ou encres. Ceci est essentiellement causé par un taux de réticulation (pouvant s'exprimer par une densité de noeuds de réticulation par unité de poids) trop élevé et le retrait lié au grand nombre d'insaturations ayant réagi. D'autre part, ces monomères sont basés sur des polyols multifonctionnels spécifiques tels que les diéthers de polyols de plus faible fonctionnalité, par exemple le ditriméthylol propane (DiTMP) ou le dipentaérythritol (DiPE), ces produits étant difficiles d'accès et avec un coût multiple de celui des polyols de départ, par exemple pour le DiTMP par rapport au triméthylol propane (TMP) ou pour le DiPE par rapport au pentaérythritol (PE). On cherche donc une solution pratique, plus simple et moins coûteuse et donc en partant des polyols de départ tels que le TMP ou le PE, laquelle solution doit remédier en même temps aux problèmes et inconvénients techniques constatés ci-haut avec les produits existants.

Le recours possible à l'alcoxylation desdits polyols de départ qui permettrait de réduire la densité de réticulation des produits obtenus, entraîne d'autre part une perte de réactivité, ce qui n'est pas acceptable car la réactivité est une des propriétés essentielles exigées, sinon la propriété essentielle de ces monomères.

La solution de la présente invention remédie à ces inconvénients avec des nouveaux produits acrylés ayant des fonctionnalités élevée sans utiliser des matières premières sophistiquées et coûteuses tels que des polyéthers ou des structures dendrimères, mais seulement en partant de polyols de base couramment utilisés en chimie et en garantissant une densité de réticulation suffisante pour les produits finaux obtenus sans être trop élevée et avec un retrait significativement plus faible, avec un compromis entre dureté et flexibilité comme définie ci-haut et une adhérence nettement améliorés.

La solution de la présente invention, tel que définie dans les revendications, consiste en un produit acrylé qui est un mélange de produits comprenant des polyéther-esters hyperbranchés de structure et de composition contrôlée à partir de polyols courants et de l'acide acrylique avec une fonctionnalité moyenne élevée et parfaitement contrôlée en acrylates par superposition de réactions successives d'estérification et d'éthérification par addition de Michael. Des structures hyperbranchées de haute fonctionnalité y sont présentes, lesquelles par l'allongement suffisant via éthérification par addition de Michael permettent à la fois une fonctionnalité élevée et une densité de réticulation suffisante sans retrait particulier, ni problème d'adhérence ou de compromis dureté/flexibilité. L'allongement par éthérification (par addition de Michael) est contrôlé par le rapport molaire en défaut d'acide acrylique par rapport aux groupements OH dudit polyol.

Parmi les avantages de cette solution par rapport à l'état de l'art antérieur, on peut citer les suivants :

- très bon contrôle d'une structure hyperbranchée polyéther-ester acrylate (PEEA) de faible viscosité, de faible hydrophilie (la quasi-totalité des groupes hydroxyles sont consommés), de très haute fonctionnalité tout en gardant une densité de double liaison modérée,
- cette structure permet l'obtention de films photoréticulés de haute flexibilité sans perdre de dureté,
- ces produits présentent une viscosité très inférieure à celle obtenue par simple polyestérification (ajout d'un diacide) ou par la polyéthérification par simple déshydratation,
- un autre avantage particulier et important est leur synthèse très simple et très pratique qui ne nécessite qu'une seule étape, en partant d'un mélange réactif d'un polyol ou un mélange de polyols courants avec l'acide acrylique en défaut stoechiométrique, comme seuls réactants et avec une catalyse acide et un reflux hétéroazéotropique pour extraire l'eau d'estérification et sans aucun besoin de séparation/purification du produit final. Contrairement aux produits courants, le produit n'est pas lavé mais neutralisé, d'où une meilleure empreinte carbone par réduction des effluents (rendement limité par la seule perte de l'eau d'estérification),

- l'indice d'hydroxyle final est très faible, d'où une faible hydrophilie par rapport à la tolérance à l'eau ou une grande hydrophobie, ce qui minimise l'impact sur l'environnement.

Parmi les autres avantages de la solution selon la présente invention, on peut citer en particulier le fait que le produit selon l'invention est un mélange de produits de structure et de composition bien contrôlée et reproductible, obtenu en une seule étape, utilisable directement comme tel pour l'application finale, sans nécessiter d'opérations coûteuses de séparation de sous-produits. Un avantage particulier de ce produit final est le fait qu'il présente une distribution moléculaire avec présence contrôlée du monomère acrylé de départ, qui joue le rôle de diluant réactif pour la composition d'application. Par conséquent, le produit final ne nécessite pas, en général, d'addition de diluant réactif supplémentaire pour ajuster sa viscosité. Par contre, il est possible d'utiliser un tel diluant réactif supplémentaire pour les masses moléculaires moyennes du produit final les plus élevées, ceci en fonction de l'application finale et de la viscosité d'application requise. Un avantage particulier desdits produits de l'invention est leur faible retrait volumique malgré leur fonctionnalité élevée en acrylates.

L'invention concerne d'abord un produit acrylé, qui est en fait un monomère ou oligomère acrylique multifonctionnel, lequel est le produit de réaction de l'acide acrylique en défaut avec un polyol multifonctionnel avec obtention d'un mélange de monomères et d'oligomères multifonctionnels acrylés, par réactions à la fois d'estérification et d'éthérification via addition de Michael sur la double liaison acrylate des groupements hydroxyles en excès portés par les esters acryliques obtenus.

Le deuxième objet de l'invention concerne un procédé d'obtention dudit produit acrylé en tant que mélange de monomères et d'oligomères acryliques multifonctionnels.

Un autre objet couvert par la présente invention concerne une composition réticulable comprenant au moins un produit acrylé tel que défini selon la présente invention.

Ensuite, l'invention couvre également l'utilisation desdits produits acrylés comme un mélange ou composition de monomères/oligomères acryliques multifonctionnels dans des compositions réticulables avec une haute densité de réticulation et un faible retrait, en particulier pour des compositions de revêtements pigmentés ou non, en particulier des peintures, vernis, encres, adhésifs ou des compositions de moulage, d'étanchéité ou de composite ou de scellement chimique.

Finalement, l'invention couvre des produits finis obtenus par l'utilisation d'un produit acrylé selon l'invention ou par réticulation d'une composition réticulable de l'invention (comprenant ledit produit acrylé), lesdits produits étant sélectionnés parmi : des revêtements pigmentés ou non, en particulier des peintures, vernis, encres, adhésifs ou des compositions de moulage, d'étanchéité ou de composite ou de scellement chimique.

Donc, le premier objet de la présente invention concerne un produit acrylé multifonctionnel, en particulier mélange ou composition de monomères et oligomères multifonctionnels acrylés, lequel produit a une fonctionnalité moyenne f en groupements acrylates par mole dudit produit supérieure à 3, de préférence entre 3 et 14 et plus préférentiellement de 4 à 14 et une densité desdits groupements allant de 4 à 12 mmoles par g dudit produit, lequel produit acrylé est le produit de réaction par estérification et d'éthérification via réaction d'addition de Michael, entre au moins un polyol $R(OH)_m$ de fonctionnalité m d'au moins 3, de préférence de 3 à 6, plus préférentiellement de 4 à 6 et l'acide acrylique ($R_1OH$), avec les groupements carboxy de l'acide acrylique étant en défaut par rapport aux groupements hydroxy dudit polyol et lequel produit final comprend dans sa composition de produit final, au moins les 3 acrylates définis selon la formule générale suivante (I) et correspondant à :
n = 0, n = 1 et n = 2 (présence obligatoire d'au moins ces 3 produits acrylés dans ledit produit acrylé) :

$$(R_1O)\text{-}[\text{-}R[OR_1]_{m\text{-}2}\text{-}O\text{-}(C=O)\text{-}CH_2\text{-}CH_2\text{-}O\text{-}]_n\text{-}R\text{-}(OR1)_{m\text{-}1} \qquad (I)$$

avec $R_1$ étant le radical acryloyle ($\text{-}(C=O)\text{-}CH=CH_2$), R étant le radical résiduel dudit polyol $R(OH)_m$ et n est le nombre d'unités répétitives éther-ester obtenues par addition de Michael, des OH résiduels des esters acrylates hydroxylés formés par estérification partielle dudit polyol (par addition de Michael) sur les groupements acrylates desdits esters acrylates et en ce que ledit produit est susceptible d'être obtenu par réactions simultanées ou successives et alternées d'estérification et d'éthérification entre l'acide acrylique ($R_1OH$) et au moins un polyol $R(OH)_m$ de fonctionnalité m d'au moins 3, de préférence de 3 à 6, avec un rapport global $r = CO_2H/OH$, allant d'une valeur supérieure à (m-1)/m et jusqu'à 0,95.

Selon une option plus particulière de l'invention, ledit produit comprend en plus un acrylate de formule générale (I) correspondant à n = 3. Cela signifie qu'au moins 4 produits acrylés de formule indiquée et avec n indiqués sont présents dans la composition dudit produit, lequel produit est ledit mélange de monomères et d'oligomères de formule selon (I). Encore plus particulièrement, ledit produit comprend en plus un acrylate de formule générale (I) correspondant à n = 4, ce qui signifie qu'au moins les produits acrylés de formule (I) indiquée, monomères et oligomères correspondant à n = 0 et n = 1 et n = 2 et n = 3 et n = 4 sont présents. Dans la présente invention, on considérera comme « monomères » les produits avec n = 0 et 1 et « oligomères » ceux avec n d'au moins 1.

Comme polyols convenables pouvant être utilisés pour la préparation du produit défini selon la présente invention, on peut citer des polyols ou des mélanges de polyols de fonctionnalité d'au moins 3, de préférence allant de 3 à 6 et plus préférentiellement de 4 à 6.

Selon une variante, le produit de l'invention est un mélange de produits de formule générale (I) avec une distribution moléculaire telle que pour 80% en poids de ladite distribution n varie de 0 à 4 et pour moins de 20% de ladite distribution n est supérieur à 4, de préférence avec une masse moyenne en nombre correspondante Mn allant de 300 à 3000 et plus préférentiellement de 300 à 2500 Daltons. Toutes les valeurs Mn données par la suite sont en Daltons.

Selon une option préférée, ledit produit est obtenu à partir d'un polyol ayant une fonctionnalité d'au moins 4 et il comprend des produits linéaires selon la formule générale (I) et en plus, au moins un produit de structure ramifiée (ou à chaîne ramifiée), de préférence hyperbranchée.

A noter qu'une structure ramifiée selon l'invention est une structure comprenant au moins un greffon latéral sur la chaîne principale, les deux, greffon et chaîne principale, étant de même nature. Une structure hyperbranchée selon la présente invention est une structure ayant par molécule au moins 12 greffons d'ordre 2 ou de deuxième génération, portés par des greffons d'ordre 1 ou de première génération portés par la chaîne principale.

Plus particulièrement, le produit selon l'invention est susceptible d'être obtenu par réactions simultanées ou successives et alternées d'estérification et d'éthérification, entre a moles d'acide acrylique ($R_1OH$) et b moles d'au moins un polyol $R(OH)_m$ de fonctionnalité m d'au moins 3, de préférence de 3 à 6, avec un rapport global r en équivalents r = $a/(m*b) = CO_2H/OH$ allant d'une valeur de 1,02*(m-1)/m à 0,95, préférentiellement de 1,035*(m-1)/m à 0,95. Ledit polyol peut être sélectionné parmi des monomères polyols et/ou des oligomères polyols et dans ce dernier cas (oligomères polyols) avec une masse moyenne en nombre Mn ne dépassant pas 600, de préférence ne dépassant pas 400. Un monomère polyol convenable pour l'invention peut être sélectionné parmi : pentaérythritol (PET), triméthylol propane (TMP), pentaérythritol alcoxylé, triméthylol propane alcoxylé, glycérol alcoxylé, sorbitol, érythritol, xylitol, de préférence pentaérythritol, triméthylol propane, pentaérythritol alcoxylé, triméthylol propane alcoxylé, sorbitol. De préférence, quand ledit polyol est alcoxylé, il y a de 1 à 4 unités alcoxy. Un oligomère polyol convenable peut être sélectionné parmi : polyéther polyols, polyester polyols, oligomères acryliques hydroxylés. Les oligomères acryliques hydroxylés peuvent être par exemple des oligomères copolymères à base d'hydroxy alkyl (méth)acrylates, avec le taux dudit hydroxy alkyl (méth)acrylate fixant la fonctionnalité dudit oligomère.

Ledit produit de l'invention peut être obtenu à partir d'un mélange de polyols comme définis ci-haut. Plus particulièrement, en plus dudit polyol de fonctionnalité d'au moins 3, est présent un deuxième polyol différent du premier et de fonctionnalité d'au moins 2. Cette option permet ainsi d'ajuster la fonctionnalité moyenne f et la compatibilité du liant avec les autres composants en fonction de l'application finale.

La longueur de chaîne dudit produit acrylé selon l'invention est caractérisée par l'indice n, lequel correspond au nombre de motifs éther-ester enchaînés, par réactions successives d'addition de Michael, d'un OH dudit polyol sur l'acide acrylique, suivie de l'estérification d'un OH résiduel (parmi m-1) dudit polyol par une autre molécule d'acide acrylique dont l'insaturation peut à nouveau faire l'objet d'une autre addition de Michael par un OH d'une autre molécule dudit polyol. Un indice n moyen peut être estimé pour une conversion totale à partir du rapport en équivalents r = $CO_2H/OH$ et la fonctionnalité m dudit polyol par la relation suivante :

$$n = [(1-r) / (r-1+1/m)]$$

A partir de n moyen, il est possible de calculer une masse moléculaire moyenne en nombre Mn compte tenu du poids moléculaire (pm) des unités répétitives et de la formule I décrite ci-haut.

De même, une fonctionnalité moyenne en groupements acrylates par produit acrylé peut être estimée (calculée) à partir de n moyen décrit ci-haut la fonctionnalité m dudit polyol et la formule I. A noter que dans le cas d'un mélange de 2 polyols de fonctionnalités m1 et m2 à des taux molaires x1 et x2 (x1 + x2 = 1) respectivement dans ledit mélange, dans ce cas, la fonctionnalité m à utiliser est la moyenne en nombre (molaire) des deux polyols selon la relation suivante :

$$m \text{ moyen} = x1*m1 + x2*m2$$

Dans le cas d'un mélange de plusieurs polyols i de fonctionnalité $m_i$ et de taux molaires $x_i$ ($\sum_i x_i = 1$), la fonctionnalité moyenne m sera égale à $m = \sum_i x_i * m_i$.

De préférence, n moyen peut varier de 0,3 à 12 et plus préférentiellement de 0,35 à 10.

Le deuxième objet de la présente invention concerne un procédé de préparation d'un produit selon l'invention comme décrit ci-haut, lequel procédé comprend les étapes suivantes :

i) le mélange dans un réacteur de l'acide acrylique et dudit polyol dans des proportions telles que le rapport global

en équivalent r = $CO_2$H/OH soit dans une plage allant d'une valeur supérieure à m-1/m et jusqu'à 0,95, de préférence de 1,02*(m-1)/m à 0,95, plus préférentiellement de 1,035*(m-1)/m à 0,95, en présence d'un catalyseur acide d'estérification et d'un solvant formant un azéotrope avec l'eau, pour former le mélange réactionnel, suivi de

ii) la mise sous reflux dudit mélange réactionnel, avec réactions simultanées ou successives et alternées d'estérification, par réaction de l'acide acrylique avec un hydroxy dudit polyol et d'éthérification, par réaction d'addition de Michael d'un hydroxy dudit polyol sur un groupement acrylate formé et élimination progressive de l'eau d'estérification, avec

iii) poursuite de la réaction jusqu'à consommation complète des fonctions OH par addition de Michael et

iv) neutralisation dudit catalyseur acide avant récupération dudit produit final, par élimination dudit solvant, sans autre étape spécifique de purification requise.

Le solvant formant un azéotrope avec l'eau est sélectionné en particulier parmi l'heptane ou le toluène ou un mélange de ces solvants dans un rapport volumique allant de 25/75 à 75/25. Le taux en poids du solvant dans le mélange réactionnel varie de 10 à 40%. La plage de reflux de l'étape ii) se situe de préférence de 95 à 130°C, plus préférentiellement de 100 à 120°C. Comme catalyseur acide d'estérification peuvent être utilisés des acides tels que l'acide méthane sulfonique (AMS) ou le p-toluène sulfonique (APTS), $H_2SO_4$ de préférence l'acide p-toluène sulfonique. De préférence, le taux de catalyseur va de 1 à 10% plus préférentiellement de 2 à 5% en poids par rapport audit polyol. La consommation des groupements (ou fonctions) OH peut être contrôlée par mesure de l'indice OH du produit fini. De même, la consommation des groupements $CO_2$H peut être contrôlée par la mesure de l'indice d'acide dudit produit. De préférence, dans le produit final plus de 95% des OH de départ sont consommés ou convertis sous forme d'ester et d'éther. De préférence, l'indice OH correspondant au produit final est inférieur à 6 mg KOH/g comme l'indice d'acide qui reste également inférieur à 6 mg KOH/g. La neutralisation du catalyseur acide est réalisée par un agent neutralisant organique soluble, comme par exemple, par une amine, telle que tertiaire ou secondaire ou par un agent neutralisant minéral solide par exemple comme le carbonate de sodium, sous forme de lit neutralisant sur lequel le mélange réactionnel peut être filtré.

A noter, en particulier, l'avantage de ce procédé qui permet par un mélange réactionnel unique (acide acrylique et polyol ou mélange de polyols) et réglage simple du rapport r = $CO_2$/OH, d'avoir un produit final acrylé multifonctionnel, ayant une distribution moléculaire bien définie et contrôlée, avec usage dudit produit tel qu'obtenu, sans aucun besoin de séparation ou de purification du produit final, ni même besoin contraignant de dilution et d'ajustement de la viscosité pour l'application finale. Ce dernier avantage est dû à la présence du monomère correspondant à n = 0 dans ledit produit, en jouant le rôle interne de diluant réactif pour l'application finale. Ceci démontre de manière supplémentaire les avantages dudit procédé et de la présente invention en général.

Un autre objet de la présente invention concerne une composition réticulable qui comprend au moins un produit tel que décrit ci-haut ou obtenu par un procédé tel que défini ci-haut selon l'invention.

Cette composition peut comprendre en plus dudit produit acrylé de l'invention, en particulier dans le cas où la Mn est supérieure à 1000 et de préférence supérieure à 1500, au moins un diluant réactif sélectionné parmi les monomères acryliques de préférence multifonctionnels. Le rôle essentiel de ce diluant, s'il y a besoin, est d'ajuster la viscosité en fonction de l'application finale.

Plus particulièrement, ladite composition est réticulable par rayonnement, en particulier sous UV en présence d'un système photoamorceur ou par faisceau d'électrons (EB) et en l'absence dans ce cas de système photoamorceur et/ou par un système d'amorçage radicalaire thermique, en particulier par système d'amorçage peroxyde (P-cure) et/ou par addition de Michael (M-cure) ou par un système mixte, en particulier par réticulation duale (dual cure) où les deux systèmes précités sont présents.

Ladite composition réticulable est plus particulièrement une composition de revêtements pigmentés ou non, de préférence choisis parmi : peinture, vernis, encre, adhésif et revêtement de gel (« gel coat ») ou une composition pour objets en 3D (tridimensionnels) par couches successives ou une composition de moulage ou une composition d'étanchéité ou une composition de composite ou une composition de scellement chimique.

L'invention couvre également l'utilisation dudit produit tel que décrit ci-haut ou obtenu par un procédé tel que défini selon l'invention dans des compositions réticulables, en particulier présentant un faible taux de retrait. Plus particulièrement, une telle utilisation s'applique à des compositions de revêtements pigmentés ou non, en particulier des peintures, vernis, encres, adhésifs et revêtements de gel (« gel coats ») ou des compositions pour objets en 3D (tridimensionnels) par couches successives ou des compositions de moulage, d'étanchéité ou de composite ou de scellement chimique.

Finalement, l'invention concerne aussi les produits finis obtenus, qui résultent de l'utilisation d'au moins un produit tel que défini ci-haut ou obtenu par un procédé tel que défini ci-haut selon l'invention ou de la réticulation d'une composition réticulable comme définie plus haut selon l'invention et de préférence produits finis choisis parmi des revêtements pigmentés ou non pigmentés, en particulier parmi les peintures, vernis, encres, adhésifs et les revêtements de gel (« gel coats») ou des objets en 3D (tridimensionnels) par couches successives ou des pièces moulées ou des joints d'étanchéité ou des composites ou des scellements chimiques.

EP 2 999 690 B1

Les exemples qui suivent sont présentés au titre d'illustration de l'invention et de ses performances et ne limitent nullement la couverture de l'invention.

EXEMPLES

1) Matières premières utilisées (voir tableau 1)

Tableau 1 : matières premières utilisées

| Nom commercial (REF) | Nom chimique | Nom abrégé | Fournisseur | Fonction selon l'invention | Fonctionnalité |
|---|---|---|---|---|---|
| Penta radcure | Pentaérythritol | PET | Perstorp | Polyol | 4 |
| TMP (Hydro) flakes | Triméthylol Propane | TMP | BASF | Polyol | 3 |
| Acide acrylique glacial | Acide acrylique | AA | Arkema | Acide acrylique | 1 |
| Toluène | Toluène | Tol | TOTAL | Solvant | |
| MSA E-pure | Acide méthane sulfonique | AMS | Arkema | Catalyseur | |
| Hydroquinone extra pure | Hydroquinone | HQ | Rhodia | Inhibiteur | |
| Paraméthoxy Phénol écailles | Ether Méthylique d'Hydroquinone | EMHQ | Rhodia | Inhibiteur | |
| CN131B | Epoxyacrylate | O-1 | Sartomer | Oligomère acrylate | |
| Darocur® 1173 | 2-Hydroxy-2-Methyl-Phenyl-Propane-1-one | PI-1 | BASF | Photo-Initiateur | |
| Dipropylamine | Dipropylamine | | BASF | Neutralisant | |
| SR 399 | Dipentaérythritol hexaacrylate | | Sartomer | Produit acrylé de référence vs l'état de l'art | 6 |

2) Préparation des produits selon l'invention

2.1) Mode opératoire pour exemples selon l'invention (exemples 1 à 4)

Le rapport r cité ci-dessus dans les exemples correspond au rapport r d'équivalents $CO_2H/OH$.

**Exemple 1** : PET avec r = COOH/OH = 0,93 (invention)

Dans un réacteur de 1 litre équipé d'un agitateur à ancre et surmonté d'un florentin avec son réfrigérant (dispositif permettant le soutirage continu de l'eau d'estérification sous reflux de solvant), d'une entrée d'air (barbotage d'air) et d'une sonde thermométrique, on introduit : 450,35 g d'acide acrylique (AA) (6,2263 mole), 228,36 g de pentaérythritol (PET) (1,6714 mole), 301,38 g de toluène, 13,83 g d'acide méthane sulfonique en solution aqueuse à 70% (AMS-aq) (0,1004 mole), 4,50 g d'hydroquinone (HQ), 1,58 g d'éther méthylique d'hydroquinone (EMHQ).
Le mélange réactionnel est mis sous reflux pendant 14 heures, passant ainsi d'une température de 100°C (début d'ébullition) à 117°C au moment de l'arrêt de l'estérification effectué par refroidissement du milieu réactionnel, après avoir distillé 117,5 ml d'eau.
Cette phase organique est neutralisée à 50°C de 17,34 g de dipropylamine (0,1864 mole) et sous agitation pendant 1 h avant distillation sous vide à 80 - 95°C et 200 - 100 millibars jusqu'à élimination complète du solvant (avec toluène résiduel < 0,1%).
On obtient un produit Poly(éther-ester) polyacrylate A-1 ayant les caractéristiques suivantes :

6

| | |
|---|---|
| Aspect : | limpide |
| Turbidité : | 4% |
| Viscosité à 25°C : | 7,4 Pa.s |
| Acidité résiduelle ou indice d'acide du produit : | 4,6 mg KOH/g |
| Indice OH du produit : | 0,73 mg KOH/g |

On réalise, par simple mélange à froid, une formulation F-1 selon la composition centésimale suivante :

A-1 : 20%

O-1 : 76%

PI-1 : 4%

Caractéristiques de la formulation F-1 :

| | |
|---|---|
| Réactivité : | 15 m/min |
| Dureté Persoz : | 77 s |
| Flexibilité : | 4 mm |
| Résistance à l'acétone : | > 300 s |

**Exemple 2** : PET mais avec r = 0,85 (invention)

467,45 g d'acide acrylique (AA) (6,4924 mole), 259,34 g de pentaérythritol (PET) (1,9069 mole), 251,19 g de toluène, 15,71 g d'acide méthane sulfonique en solution aqueuse à 70% (AMS-aq) (0,1145 mole), 4,67 g d'hydroquinone (HQ), 1,64 g d'éther méthylique d'hydroquinone (EMHQ).

Le mélange réactionnel est mis sous reflux pendant 19 heures, passant ainsi d'une température de 100°C (début d'ébullition) à 120°C au moment de l'arrêt de l'estérification effectué par refroidissement du milieu réactionnel, après avoir distillé 122 ml d'eau. La neutralisation de la phase organique et l'élimination (distillation) du solvant et récupération du produit fini est similaire à celle décrite pour l'exemple 1.

On obtient un produit Poly(éther-ester) polyacrylate A-2 ayant les caractéristiques suivantes :

| | |
|---|---|
| Aspect : | limpide |
| Turbidité : | 5,5% |
| Viscosité à 25°C : | 72 Pa.s |
| Acidité résiduelle (indice d'acide) : | 1,3 mg KOH/g |
| Indice OH du produit : | < 5 |

**Exemple 3** : TMP avec r = 0,75 (invention)

372,02 g d'acide acrylique (AA) (5,1669 mole), 307,65 g de triméthylolepropane (TMP) (2,2959 mole), 301,14 g de toluène, 14,17 g d'acide méthane sulfonique en solution aqueuse à 70% (AMS-aq) (0,1033 mole), 3,72 g d'hydroquinone (HQ), 1,30 g d'éther méthylique d'hydroquinone (EMHQ).

Le mélange réactionnel est mis sous reflux pendant 13 heures, passant ainsi d'une température de 100°C (début d'ébullition) à 120°C au moment de l'arrêt de l'estérification effectué par refroidissement du milieu réactionnel, après avoir distillé 98 ml d'eau. La neutralisation de la phase organique et l'élimination (distillation) du solvant et récupération du produit fini est similaire à celle décrite pour l'exemple 1.

On obtient un produit Poly(éther-ester)polyacrylate A-3 ayant les caractéristiques suivantes :

| | |
|---|---|
| Aspect : | limpide |
| Turbidité : | 4,2% |
| Viscosité à 25°C : | 8,2 Pa.s |
| Acidité résiduelle (indice d'acide) : | 3,3 mg KOH/g |

(suite)

| | |
|---|---|
| Indice OH du produit : | < 5 |

**Exemple 4** : TMP avec r = 0,70 (invention)

360,19 g d'acide acrylique (AA) (5,0026 mole), 319,15 g de triméthylolepropane (TMP) (2,3817 mole), 301,11 g de toluène, 14,70 g d'acide méthane sulfonique en solution aqueuse à 70% (AMS-aq) (0,1072 mole), 3,60 g d'hydroquinone (HQ), 1,26 g d'Ether Méthylique d'hydroquinone (EMHQ).

Le mélange réactionnel est mis sous reflux pendant 15 heures, passant ainsi d'une température de 100°C (début d'ébullition) à 119°C au moment de l'arrêt de l'estérification effectué par refroidissement du milieu réactionnel, après avoir distillé 97,5 ml d'eau. La neutralisation de la phase organique et l'élimination (distillation) du solvant et récupération du produit fini est similaire à celle décrite pour l'exemple 1.

On obtient un produit Poly(éther-ester)polyacrylate A-4 ayant les caractéristiques suivantes :

| | |
|---|---|
| Aspect : | limpide |
| Turbidité : | 4,6% |
| Viscosité à 25°C : | 68 Pa.s |
| Acidité résiduelle (indice d'acide) : | 3,2 mg KOH/g |
| Indice OH du produit : | < 5 |

**Exemple 5** : mélange PET + TMP, r = 0,92 (invention)

462,15 g d'acide acrylique (AA) (6,4188 mole), 189,69 g de pentaérythritol (PET) (1,3948 mole), 62,30 g de TMP (0,4649 moles), 264,36 g de toluène, 15,25 g d'acide méthane sulfonique en solution aqueuse à 70% (AMS-aq) (0,1112 mole), 4,62 g d'hydroquinone (HQ), 1,62 g d'éther méthylique d'hydroquinone (EMHQ).

Le mélange réactionnel est mis sous reflux pendant 20 heures, passant ainsi d'une température de 100°C (début d'ébullition) à 120°C au moment de l'arrêt de l'estérification effectué par refroidissement du milieu réactionnel, après avoir distillé 80 ml d'eau. La neutralisation de la phase organique et l'élimination (distillation) du solvant et récupération du produit fini est similaire à celle décrite pour l'exemple 1.

On obtient un produit Poly(éther-ester)polyacrylate A-5 ayant les caractéristiques suivantes :

| | |
|---|---|
| Aspect : | limpide |
| Turbidité : | 8,7% |
| Viscosité à 25°C : | 2,6 Pa.s |
| Acidité résiduelle (indice d'acide) : | 5,1 mg KOH/g |
| Indice OH du produit : | < 5 |

**Exemple 6 :** mélange PET + TMP, r = 0,75 (invention)

382,69 g d'acide acrylique (AA) (5,3152 mole), 74,04 g de pentaérythritol (PET) (0,5444 mole), 219,7 g de TMP (1,6356 moles), 301,17 g de toluène, 17,76 g d'acide méthane sulfonique en solution aqueuse à 70% (AMS-aq) (0,1295 mole), 3,83 g d'hydroquinone (HQ), 1,34 g d'éther méthylique d'hydroquinone (EMHQ).

Le mélange réactionnel est mis sous reflux pendant 11 heures, passant ainsi d'une température de 100°C (début d'ébullition) à 118°C au moment de l'arrêt de l'estérification effectué par refroidissement du milieu réactionnel, après avoir distillé 73 ml d'eau. La neutralisation de la phase organique et l'élimination (distillation) du solvant et récupération du produit fini est similaire à celle décrite pour l'exemple 1.

On obtient un produit Poly(éther-ester)polyacrylate A-6 ayant les caractéristiques suivantes :

| | |
|---|---|
| Aspect : | limpide |
| Turbidité: | 11,7% |
| Viscosité à 25°C : | 48 Pa.s |
| Acidité résiduelle (indice d'acide) : | 1,26 mg KOH/g |
| Indice OH du produit : | < 5 |

On constate dans tous ces exemples que l'eau éliminée (distillée) correspond à une estérification quasi-complète des groupements carboxy de l'AA.

Les caractéristiques moléculaires des produits obtenus sont présentées ci-dessous dans le tableau 2.

Tableau 2 : Caractéristiques moléculaires des produits préparés selon l'invention

| REF Exemple | Polyol | Fonctionnalité Polyol (ou moyenne vs mélange) | $r = CO_2/OH$ | n moyen calc | Mn Calc (g/mole) | Mn GPC | Nombre moyen acrylates par chaîne calc | Acrylates mmoles/g calc |
|---|---|---|---|---|---|---|---|---|
| A-1 | PET | 4 | 0,93 | 0,39 | 468 | 740 | 4,78 | 10,21 |
| A-2 | PET | 4 | 0,85 | 1,5 | 799 | | 7,00 | 8,76 |
| A-3 | TMP | 3 | 0,75 | 3,0 | 1022 | | 6,00 | 5,87 |
| A-4 | TMP | 3 | 0,70 | 9,0 | 2474 | | 12,00 | 4,85 |
| A-5 | PT/TMP | 3,75 | 0,93 | 0,43 | 460 | | 4,50 | 9,79 |
| A-6 | PT/TMP | 3,25 | 0,75 | 4,33 | 1271 | | 8,67 | 6,82 |

Tableau 3 : distribution en % poids selon n (par Chromatographie Liquide HPLC)

| REF Exemple | n = 0 | n = 1 | n = 2 | n = 3 | n = 4 | n > 4 |
|---|---|---|---|---|---|---|
| A-1 | 44% | 14% | 8% | 8% | 6% | 20% |

METHODES DE MESURE ET DE CARACTERISATION

Détermination de l'aspect : On observe visuellement le produit à la lumière du jour, à travers un flacon en verre blanc de 60 ml et on distingue si le produit est :

- Limpide : aucune turbidité, il est comparable à l'eau,
- Voilé : ne permettant plus une vision nette à travers le flacon,
- Trouble : flacon opaque, aucune image ne peut être perçue à travers le flacon.

Détermination de la turbidité : C'est le pourcentage de lumière diffusée par rapport à la lumière totale transmise par l'échantillon contenu dans une cuve transparente de 50 ml (60 mm X 40 mm X 20 mm). La mesure est effectuée à l'aide d'un spectro-colorimètre « Colorquest XE»® Hunterlab.

Détermination de la viscosité Noury : On mesure le temps de parcours, dans le liquide à caractériser, d'une bille d'acier soumise à sa gravité. La norme AFNOR XP.T51-213 précise en particulier la géométrie du récipient, le diamètre de la bille (2 mm) et le parcours de la bille (104 mm). Dans ces conditions, la viscosité dynamique est proportionnelle au temps de parcours de la bille avec : 1 seconde $\Leftrightarrow$ 0,1 Pa.s.

Détermination de l'acidité résiduelle : On exprime l'acidité du produit à caractériser en milligrammes de potasse équivalente par gramme de produit. Pour cela, on effectue un dosage acido-basique dans les conditions suivantes : une masse m de produit (environ exactement 10 grammes) est dissoute dans 50 ml d'un mélange toluène / éthanol (2 vol / 1 vol). Après dissolution complète, on titre avec une solution de potasse de normalité N (environ exactement 0,1 N). Le point équivalent est détecté par une électrode combinée asservissant une burette automatique (appareil de titration automatique « 716 DMS Titrino »® Metrohm) délivrant le volume équivalent $V_E$. Après réalisation d'un essai à blanc (50 ml du mélange toluène / éthanol seul) qui permet de déterminer le volume équivalent $V_B$, on calcule l'indice d'acide (IA) par la formule IA = $(V_E - V_B)$*N*56,1 / m.

Détermination de la réactivité : La formulation F### est appliquée en film de 12 $\mu$m sur une carte contraste (« Penoparc charts form 1B »® Leneta), puis est réticulé à l'aide d'une lampe Fusion Hg à 120 W/cm. On mesure la vitesse de passage minimum nécessaire (en m / min) pour obtenir un film sec au toucher.

Pour les tests suivants de dureté, flexibilité et de résistance à l'acétone, les films photoréticulés sont laissés en salle climatisée (T = 23°C) pendant 24 heures après réticulation et avant les mesures.

Détermination de la dureté Persoz : La formulation à examiner est appliquée en film de 100 $\mu$m sur une plaque en verre et réticulée par une lampe Fusion Hg 120 W/cm à une vitesse de 8 m / min.

On mesure le nombre d'oscillations avant l'amortissement des oscillations (passage de 12° à 4° d'amplitude), d'un pendule au contact de la plaque de verre revêtue d'après la norme ISO 1522.

Détermination de la flexibilité : La formulation F### est appliquée en film de 100 $\mu$m sur une plaque en acier lisse de 25/10 mm d'épaisseur (D-46®Q-Panel), puis réticulé par une lampe Fusion Hg 120 W/cm à une vitesse de 8 m / min.

On courbe la plaque revêtue sur des mandrins cylindriques d'après la norme ISO 1519. On exprime le résultat par la valeur (en mm) du rayon de courbure le plus faible qu'on peut infliger au revêtement sans qu'il ne se fissure ni se décolle du support.

Détermination de la résistance à l'acétone : La formulation F### est appliquée en film de 12 $\mu$m sur une plaque en verre, puis réticulé par une lampe Fusion Hg 120 W/cm à une vitesse de 8 m / min. On frotte le revêtement avec un chiffon imbibé d'acétone. Le résultat est le temps (exprimé en seconde) au-delà duquel le film se décolle et/ou se désagrège.

Masse moléculaire moyenne en nombre Mn : Calculée selon méthode précisée dans la description et/ou mesurée par GPC dans le THF comme solvant et Mn exprimée en équivalents polystyrène sur colonnes calibrées avec étalons polystyrène.

Acrylates par unité de poids calculé à partir du bilan matière et/ou mesuré par RMN C[13] : Distribution (%) en poids de n : par HPLC avec un détecteur UV (230 nm) barrette de diode.

Tolérance à l'eau : La mesure directe de la quantité limite d'eau au-delà de laquelle le mélange eau - produit se trouble par démixtion (à température ambiante) est peu précise. C'est pourquoi on a recours à une méthode indirecte : on mesure le point de trouble (température de démixtion eau - produit) pour 2 compositions connues (environ exactement 2 et 4% d'eau par exemple) ; puis on détermine la composition limite de démixtion par interpolation linéaire de cette valeur à température ambiante (20°C).

Retrait : Il est mesuré par le rayon de courbure d'un film standard réticulé (après réticulation) avec 3 niveaux distingués de courbure causée par le retrait (le retrait crée des tensions internes dans le film qui le font courber) avec rayon de courbure inversement croissant avec le retrait :

    1) rayon nul (0 : meilleur résultat)
    2) rayon entre 10 et 20 cm
    3) rayon < 5 cm

Résultats d'évaluation : Ces résultats sont présentés au tableau 4 ci-dessous sur des formulations.

Tableau 4 : résultats de performances des compositions des revêtements

| Toutes les formulations testées sont identiques à la composition de la formulation F-1 décrite en page 11. | | | | | | | |
|---|---|---|---|---|---|---|---|
| REF du produit acrylé dans la formulation testée → | **A-1** | **A-2** | **A-3** | **A-4** | **A-5** | **A-6** | **SR 399** |
| PERFORMANCE testée | | | | | | | |
| Réactivité (m/min) | 15 | 15 | 10 | 10 | 10 | < 5 | 25 |
| Dureté Persoz | 77 | 68 | 46 | 50 | 94 | 54 | 135 |
| Flexibilité | 4 | < 3 | < 3 | < 3 | 4 | > 3 | 10 |
| Résistance Acétone (s) | > 300 | > 300 | 65 | 60 | > 300 | 205 | > 300 |
| Retrait Courbure (cm) | 10 - 20 | 10 - 20 | 0 | 0 | 10 - 20 | 0 | < 5 |
| Tolérance vs eau (%) | 0,6 | | | | | | 1,25 |

Toutes les formulations testées sont identiques à F-1 décrite page 11, sauf pour la mesure du retrait où on utilise la formulation F-2 suivante :

- Produit acrylé selon l'invention (A-1 à A-6) et produit de référence : 96%
- PI-1 : 4%

**Revendications**

**1.** Produit acrylé multifonctionnel **caractérisé en ce qu'**il a une fonctionnalité moyenne f supérieure à 3, de préférence entre 3 et 14 et plus préférentiellement de 4 à 14 en groupements acrylates par mole dudit produit et une densité en ces groupements allant de 4 à 12 mmoles par g dudit produit, lequel est le produit de réaction par estérification et par éthérification via réaction d'addition de Michael, entre au moins un polyol $R(OH)_m$ de fonctionnalité m d'au moins 3, de préférence de 3 à 6, plus préférentiellement de 4 à 6 et l'acide acrylique ($R_1OH$), avec les groupements carboxy de l'acide acrylique étant en défaut par rapport aux groupements hydroxy dudit polyol et lequel produit final comprend dans sa composition, au moins 3 acrylates définis selon la formule générale (I) suivante et correspondant à n = 0, n = 1, n = 2 :

$$(R_1O)\text{-}[\text{-}R[OR_1]_{m\text{-}2}\text{-}O\text{-}(C{=}O)\text{-}CH_2\text{-}CH_2\text{-}O\text{-}]_n\text{-}R\text{-}(OR1)_{m\text{-}1} \qquad (I)$$

avec $R_1$ étant le radical acryloyle ($\text{-}(C{=}O)\text{-}CH{=}CH_2$), R étant le radical résiduel dudit polyol $R(OH)_m$ et n est le nombre d'unités répétitives éther-ester obtenues par addition de Michael, des OH résiduels des esters acrylates hydroxylés formés par estérification partielle dudit polyol (par addition de Michael) sur les groupements acrylates desdits esters acrylates et **en ce que** ledit produit est susceptible d'être obtenu par réactions simultanées ou successives et alternées d'estérification et d'éthérification entre l'acide acrylique ($R_1OH$) et au moins un polyol $R(OH)_m$ de fonctionnalité m d'au moins 3, de préférence de 3 à 6, avec un rapport global $r = CO_2H/OH$, allant d'une valeur supérieure à (m-1)/m et jusqu'à 0,95.

2. Produit selon la revendication 1, **caractérisé en ce qu'**il comprend en plus, un acrylate de formule générale (I) correspondant à n = 3.

3. Produit selon la revendication 2, **caractérisé en ce qu'**il comprend en plus un acrylate de formule générale (I) correspondant à n = 4.

4. Produit selon l'une des revendication 1 à 3, **caractérisé en ce qu'**il est un mélange de produits de formule générale (I) avec une distribution moléculaire telle que, pour au moins 80% en poids de ladite distribution n varie de 0 à 4 et pour moins de 20% de ladite distribution n est supérieur à 4, de préférence avec une masse moyenne en nombre correspondante Mn allant de 300 à 3000 et plus préférentiellement de 300 à 2500.

5. Produit selon l'une des revendications 1 à 4, **caractérisé en ce que** ledit polyol a une fonctionnalité d'au moins 4 et que ledit produit comprend des produits linéaires selon la formule générale (I) et en plus au moins un produit de structure ramifiée (ou à chaîne ramifiée), de préférence hyperbranchée.

6. Produit selon l'une des revendications 1 à 5, **caractérisé en ce que** ledit rapport global $r = CO_2H/OH$ va de 1,02*(m-1)/m à 0,95 et de préférence de 1,035*(m-1)/m à 0,95.

7. Produit selon l'une des revendications 1 à 6, **caractérisé en ce que** ledit polyol est sélectionné parmi des monomères polyols et/ou des oligomères polyols de Mn (oligomères polyols) ne dépassant pas 600, de préférence ne dépassant pas 400.

8. Produit selon la revendication 7, **caractérisé en ce que** ledit polyol est un monomère polyol et sélectionné parmi : pentaérythritol, triméthylol propane, pentaérythritol alcoxylé, triméthylol propane alcoxylé, glycérol alcoxylé, sorbitol, érythritol, xylitol, de préférence pentaérythritol, triméthylol propane, pentaérythritol alcoxylé, triméthylol propane alcoxylé, sorbitol.

9. Produit selon la revendication 7, **caractérisé en ce que** ledit polyol est un oligomère polyol parmi polyéther polyols, polyester polyols, oligomères acryliques hydroxylés.

10. Produit selon l'une des revendications 1 à 8, **caractérisé en ce qu'**en plus dudit polyol de fonctionnalité d'au moins 3, est présent un deuxième polyol différent du premier de fonctionnalité d'au moins 2.

11. Procédé de préparation d'un produit tel que défini selon l'une des revendications 1 à 10, **caractérisé en ce qu'**il comprend :

i) le mélange dans un réacteur de l'acide acrylique et dudit polyol dans des proportions telles que le rapport molaire global $r = CO_2H/OH$ soit dans une plage allant d'une valeur supérieure à (m-1)/m et jusqu'à 0,95, de préférence de 1,02*(m-1)/m à 0,95 et plus préférentiellement de 1,035*(m-1)/m à 0,95, en présence d'un catalyseur acide d'estérification et d'un solvant formant un azéotrope avec l'eau, pour former le mélange réactionnel, suivi de

ii) la mise sous reflux dudit mélange réactionnel, avec réactions simultanées ou successives et alternées d'estérification, par réaction de l'acide acrylique avec un hydroxy dudit polyol et d'éthérification par réaction d'addition de Michael d'un hydroxy dudit polyol sur un groupement acrylate formé et élimination progressive de l'eau d'estérification, avec

iii) poursuite de la réaction jusqu'à consommation complète des fonctions OH par addition de Michael et

iv) neutralisation dudit catalyseur acide avant récupération dudit produit final par élimination dudit solvant, sans autre étape spécifique de purification requise.

**12.** Composition réticulable **caractérisée en ce qu'**elle comprend au moins un produit tel que défini selon l'une des revendications 1 à 10 ou obtenu par un procédé tel que défini selon la revendication 11.

**13.** Composition selon la revendication 12, **caractérisée en ce que** pour Mn supérieure à 1000, de préférence supérieure à 1500, elle comprend en plus, au moins un diluant réactif, sélectionné parmi les monomères acryliques de préférence multifonctionnels.

**14.** Composition selon la revendication 12 ou 13, **caractérisée en ce qu'**elle est une composition réticulable par rayonnement, en particulier UV en présence d'un système photoamorceur ou par faisceau d'électrons (EB) et en l'absence de système photoamorceur et/ou par un système d'amorçage radicalaire thermique, en particulier par système d'amorçage peroxyde (P-cure) et/ou par addition de Michael (M-cure) ou un système mixte, en particulier par réticulation duale (dual cure).

**15.** Composition selon l'une des revendications 12 à 14, **caractérisée en ce qu'**il s'agit d'une composition de revêtements pigmentés ou non, de préférence parmi peinture, vernis, encre, adhésif et revêtement de gel (« gel coat ») ou d'une composition pour objets en 3D (tridimensionnels) par couches successives ou d'une composition de moulage ou d'une composition d'étanchéité ou d'une composition de composite ou d'une composition de scellement chimique.

**16.** Utilisation d'un produit tel que défini selon l'une des revendications 1 à 10 ou obtenu par un procédé tel que défini selon la revendication 11, dans des compositions réticulables, en particulier présentant un faible taux de retrait.

**17.** Utilisation selon la revendication 16, **caractérisée en ce qu'**elle s'applique à des compositions de revêtements pigmentés ou non, en particulier des peintures, vernis, encres, adhésifs et revêtements de gel (« gel coats ») ou des compositions pour objets en 3D (tridimensionnels) par couches successives ou des compositions de moulage, d'étanchéité ou de composite ou de scellement chimique.

**18.** Produits finis obtenus, **caractérisés en ce qu'**ils résultent de l'utilisation d'au moins un produit tel que défini selon l'une des revendications 1 à 10 ou obtenu par un procédé tel que défini selon la revendication 11 ou de la réticulation d'une composition telle que définie selon l'une des revendications 12 à 15 de préférence choisis parmi des revêtements pigmentés ou non pigmentés, en particulier parmi les peintures, vernis, encres, adhésifs et les revêtements de gel (« gel coats ») ou des objets en 3D (tridimensionnels) par couches successives ou des pièces moulées ou des joints d'étanchéité ou des composites ou des scellements chimiques.

**Patentansprüche**

**1.** Multifunktionelles Acrylprodukt, **dadurch gekennzeichnet, dass** es eine mittlere Acrylatgruppen-Funktionalität f von mehr als 3, vorzugsweise zwischen 3 und 14 und weiter bevorzugt von 4 bis 14 pro Mol des Produkts und eine Dichte dieser Gruppen im Bereich von 4 bis 12 mmol pro g des Produkts, bei dem es sich um das Produkt der Reaktion durch Veresterung und Veretherung über eine Michael-Additionsreaktion zwischen mindestens einem Polyol $R(OH)_m$ mit einer Funktionalität m von mindestens 3, vorzugsweise von 3 bis 6 und weiter bevorzugt von 4 bis 6, und Acrylsäure ($R_1OH$) handelt, wobei die Carboxygruppen der Acrylsäure im Defizit gegenüber den Hydroxygruppen des Polyols vorliegen und das Endprodukt in seiner Zusammensetzung mindestens gemäß der folgenden allgemeinen Formel (I) definierte und n = 0, n = 1 und n = 2 entsprechende Acrylate umfasst:

$$(R_1O)\text{-}[\text{-}R[OR_1]_{m-2}\text{-}O\text{-}(C=O)\text{-}CH_2\text{-}CH_2\text{-}O\text{-}]_n\text{-}R\text{-}(OR1)_{m-1} \qquad (I)$$

wobei $R_1$ für den Acryloylrest (-(C=O)-CH=CH$_2$) steht, R für den verbleibenden Rest des Polyols $R(OH)_m$ steht und

n für die Zahl der durch Michael-Addition der restlichen OH-Gruppen der durch partielle Veresterung des Polyols (durch Michael-Addition) an den Acrylatgruppen der Acrylatester gebildeten hydroxylierten Acrylatester erhaltenen Etherester-Wiederholungseinheiten steht, und dadurch, dass das Produkt durch gleichzeitige oder aufeinanderfolgende und alternierende Veresterungs- und Veretherungsreaktionen zwischen der Acrylsäure ($R_1OH$) und mindestens einem Polyol $R(OH)_m$ mit einer Funktionalität m von mindestens 3, vorzugsweise von 3 bis 6, mit einem Gesamtverhältnis $r = CO_2H/OH$ im Bereich von einem Wert von mehr als (m-1)/m und bis 0,95 erhältlich ist.

2. Produkt nach Anspruch 1, **dadurch gekennzeichnet, dass** es außerdem ein Acrylat der allgemeinen Formel (I), das n = 3 entspricht, umfasst.

3. Produkt nach Anspruch 2, **dadurch gekennzeichnet, dass** es außerdem ein Acrylat der allgemeinen Formel (I), das n = 4 entspricht, umfasst.

4. Produkt nach einem der Ansprüche 1 bis 3, **dadurch gekennzeichnet, dass** es sich um ein Gemisch von Produkten der allgemeinen Formel (I) mit einer solchen Molekularverteilung handelt, dass für mindestens 80 Gew.-% der Verteilung n von 0 bis 4 variiert und für weniger als 20 % der Verteilung n größer als 4 ist, vorzugsweise mit einer entsprechenden zahlenmittleren Molmasse Mn im Bereich von 300 bis 3000 und weiter bevorzugt von 300 bis 2500.

5. Produkt nach einem der Ansprüche 1 bis 4, **dadurch gekennzeichnet, dass** das Polyol eine Funktionalität von mindestens 4 aufweist und dass das Produkt lineare Produkte gemäß der allgemeinen Formel (I) und außerdem mindestens ein Produkt mit verzweigter Struktur (oder verzweigter Kette), vorzugsweise hyperverzweigter Struktur, umfasst.

6. Produkt nach einem der Ansprüche 1 bis 5, **dadurch gekennzeichnet, dass** das Gesamtverhältnis $r = CO_2H/OH$ im Bereich von 1,02*(m-1)/m bis 0,95 und weiter bevorzugt von 1,035*(m-1)/m bis 0,95 liegt.

7. Produkt nach einem der Ansprüche 1 bis 6, **dadurch gekennzeichnet, dass** das Polyol aus Polyol-Monomeren und/oder Polyol-Oligomeren mit einem Mn (Polyol-Oligomere) von höchstens 600 und vorzugsweise höchstens 400 ausgewählt ist.

8. Produkt nach Anspruch 7, **dadurch gekennzeichnet, dass** das Polyol ein Polyol-Monomer ist und aus Pentaerythritol, Trimethylolpropan, alkoxyliertem Pentaerythritol, alkoxyliertem Trimethylolpropan, alkoxyliertem Glycerin, Sorbitol, Erythritol, Xylitol, vorzugsweise Pentaerythritol, Trimethylolpropan, alkoxyliertem Pentaerythritol, alkoxyliertem Trimethylolpropan, Sorbitol, ausgewählt ist.

9. Produkt nach Anspruch 7, **dadurch gekennzeichnet, dass** das Polyol ein Polyol-Oligomer ist, das aus Polyetherpolyolen, Polyesterpolyolen und hydroxylierten Acryloligomeren ausgewählt ist.

10. Produkt nach einem der Ansprüche 1 bis 8, **dadurch gekennzeichnet, dass** neben dem Polyol mit einer Funktionalität von mindestens 3 ein zweites Polyol mit einer Funktionalität von mindestens 2, das von dem ersten Polyol verschieden ist, vorliegt.

11. Verfahren zur Herstellung eines Produkts gemäß einem der Ansprüche 1 bis 10, **dadurch gekennzeichnet, dass** es Folgendes umfasst:

i) Mischen von Acrylsäure und dem Polyol in solchen Anteilen, dass das Gesamtmolverhältnis $r = CO_2H/OH$ im Bereich von mehr als (m-1)/m und bis 0,95, vorzugsweise von 1,02*(m-1)/m bis 0,95 und weiter bevorzugt von 1,035*(m-1)/m bis 0,95 liegt, in einem Reaktor in Gegenwart eines sauren Veresterungskatalysators und eines Lösungsmittels, das mit Wasser ein Azeotrop bildet, zur Bildung der Reaktionsmischung, anschließend
ii) Refluxieren der Reaktionsmischung mit gleichzeitigen oder aufeinanderfolgenden und alternierenden Veresterungsreaktionen durch Reaktion der Acrylsäure mit einer Hydroxygruppe des Polyols und Veretherungsreaktionen durch Michael-Additionsreaktion einer Hydroxygruppe des Polyols mit einer gebildeten Acrylatgruppe und allmähliches Eliminieren des Veresterungswassers, mit
iii) Fortsetzen der Reaktion bis zum vollständigen Verbrauch der OH-Funktionen durch Michael-Addition und
iv) Neutralisieren des sauren Katalysators vor der Gewinnung des Endprodukts durch Entfernung des Lösungsmittels, ohne dass ein anderer spezifischer Reinigungsschritt erforderlich ist.

12. Vernetzbare Zusammensetzung, **dadurch gekennzeichnet, dass** sie mindestens ein Produkt gemäß einem der Ansprüche 1 bis 10 oder ein durch ein Verfahren gemäß Anspruch 11 erhaltenes Produkt umfasst.

13. Zusammensetzung nach Anspruch 12, **dadurch gekennzeichnet, dass** sie für Mn größer 1000, vorzugsweise größer 1500, außerdem mindestens ein reaktives Verdünnungsmittel, das aus AcrylMonomeren, die vorzugsweise multifunktionell sind, ausgewählt ist, umfasst.

14. Zusammensetzung nach Anspruch 12 oder 13, **dadurch gekennzeichnet, dass** es sich um eine Zusammensetzung handelt, die durch Strahlung, insbesondere UV-Strahlung, in Gegenwart eines Photoinitiatorsystems und/oder mit einem Elektronenstrahl (EB) und in Abwesenheit eines Photoinitiatorsystems und/oder durch ein thermisches Radikalinitiatorsystem insbesondere durch ein Peroxid-Initiatorsystem (P-Härtung) und/oder durch Michael-Addition (M-Härtung) oder ein gemischtes System, insbesondere durch duale Vernetzung (Dualhärtung), vernetzbar ist.

15. Zusammensetzung nach einem der Ansprüche 12 bis 14, **dadurch gekennzeichnet, dass** es sich um eine pigmentierte oder unpigmentierte Beschichtungszusammensetzung handelt, die vorzugsweise aus einem Anstrichmittel, einem Lack, einer Tinte, einem Klebstoff und einem Gelcoat oder einer Zusammensetzung für 3-D-Gegenstände (dreidimensionale Gegenstände) durch aufeinanderfolgende Schichten oder einer Formmasse oder einer Dichtungsmasse oder einer Verbundzusammensetzung oder einer Zusammensetzung zur chemischen Versiegelung ausgewählt ist.

16. Verwendung eines Produkts gemäß einem der Ansprüche 1 bis 10 oder eines durch ein Verfahren gemäß Anspruch 11 hergestellten Produkts in vernetzbaren Zusammensetzungen, insbesondere mit geringem Schrumpfungsgrad.

17. Verwendung nach Anspruch 16, **dadurch gekennzeichnet, dass** sie auf pigmentierte oder unpigmentierte Beschichtungszusammensetzungen, insbesondere Anstrichmittel, Lacke, Tinten, Klebstoffe und Gelcoats oder Zusammensetzungen für schichtweise aufgebaute 3-D-Gegenstände (dreidimensionale Gegenstände) oder Formmassen, Dichtungsmassen oder Verbundzusammensetzungen oder Zusammensetzungen zur chemischen Versiegelung, anwendbar ist.

18. Erhaltene Erzeugnisse, **dadurch gekennzeichnet, dass** sie sich aus der Verwendung mindestens eines Produkts gemäß einem der Ansprüche 1 bis 10 oder eines durch ein Verfahren gemäß Anspruch 11 erhaltenen Produkts oder die Vernetzung einer Zusammensetzung gemäß einem der Ansprüche 12 bis 15 ergeben, vorzugsweise ausgewählt aus pigmentierten oder unpigmentierten Beschichtungen, insbesondere aus Anstrichmitteln, Lacken, Tinten, Klebstoffen und Gelcoats oder schichtweise aufgebauten 3-D-Gegenständen (dreidimensionalen Gegenständen) oder Formteilen oder Dichtungen oder Verbundwerkstoffen oder chemischen Versiegelungen.

## Claims

1. Multifunctional acrylic product, **characterized in that** it has a mean functionality f of acrylate groups per mole of the said product of greater than 3, preferably between 3 and 14 and more preferentially from 4 to 14 and a density of the said groups ranging from 4 to 12 mmol per g of the said product, which is the product of reaction by esterification and etherification, via Michael addition reaction, between at least one polyol $R(OH)_m$ with a functionality m of at least 3, preferably from 3 to 6 and more preferentially from 4 to 6, and acrylic acid ($R_1OH$), with the carboxyl groups of acrylic acid being in deficit relative to the hydroxyl groups of the said polyol, and which final product comprises in its composition at least three defined acrylates according to the general formula (I) below and corresponding to n = 0, n = 1, n = 2:

$$(R_1O)\text{-}[\text{-}R[OR_1]_{m-2}\text{-}O\text{-}(C=O)\text{-}CH_2\text{-}CH_2\text{-}O\text{-}]_n\text{-}R\text{-}(OR1)_{m-1} \qquad (I)$$

with $R_1$ being the acryloyl radical ($\text{-}(C=O)\text{-}CH=CH_2$), R being the residual radical of the said polyol $R(OH)_m$ and n is the number of ether-ester repeating units obtained by Michael addition of the residual OH groups of the hydroxylated acrylate esters formed by partial esterification of the said polyol (by Michael addition) on the acrylate groups of the said acrylate esters and **in that** said product may be obtained by simultaneous or successive and alternating esterification and etherification reactions between the acrylic acid (R1OH) and at least one polyol $R(OH)m$ with a functionality m of at least 3 and preferably from 3 to 6, with an overall ratio r = $CO_2H/OH$ ranging from a value greater than (m-1)/m and up to 0.95.

2. Product according to Claim 1, **characterized in that** it also comprises an acrylate of general formula (I) corresponding to n = 3.

3. Product according to Claim 2, **characterized in that** it also comprises an acrylate of general formula (I) corresponding to n = 4.

4. Product according to one of Claims 1 to 3, **characterized in that** it is a mixture of products of general formula (I) with a molecular distribution such that, for at least 80% by weight of the said distribution, n ranges from 0 to 4, and, for less than 20% of the said distribution, n is greater than 4, preferably with a corresponding number-average mass Mn ranging from 300 to 3000 and more preferentially from 300 to 2500.

5. Product according to one of Claims 1 to 4, **characterized in that** the said polyol has a functionality of at least 4 and such that the said product comprises linear products according to the general formula (I) and also at least one product of branched structure (or with a branched chain), preferably hyperbranched.

6. Product according to one of Claims 1 to 5, **characterized in that** said overall ratio r = $CO_2H/OH$ ranges from $1.02*(m-1)/m$ to 0.95 and preferably from $1.035*(m-1)/m$ to 0.95.

7. Product according to one of Claims 1 to 6, **characterized in that** the said polyol is selected from polyol monomers and polyol oligomers with an Mn (polyol oligomers) not exceeding 600 and preferably not exceeding 400.

8. Product according to Claim 7, **characterized in that** the said polyol is a polyol monomer and is selected from: pentaerythritol, trimethylolpropane, alkoxylated pentaerythritol, alkoxylated trimethylolpropane, alkoxylated glycerol, sorbitol, erythritol, xylitol, preferably pentaerythritol, trimethylolpropane, alkoxylated pentaerythritol, alkoxylated trimethylolpropane, sorbitol.

9. Product according to Claim 7, **characterized in that** the said polyol is a polyol oligomer chosen from polyether polyols, polyesterpolyols and hydroxylated acrylic oligomers.

10. Product according to one of Claims 1 to 8, **characterized in that**, in addition to the said polyol with a functionality of at least 3, there is a second polyol different from the first, with a functionality of at least 2.

11. Process for preparing a product as defined according to one of Claims 1 to 10, **characterized in that** it comprises:

> i) mixing in a reactor of acrylic acid and of the said polyol in proportions such that the overall equivalent ratio r = $CO_2H/OH$ is within a range having a value greater than (m-1)/m and up to 0.95, preferably from $1.02*(m-1)/m$ to 0.95 and more preferentially from $1.035*(m-1)/m$ to 0.95, in the presence of an acidic esterification catalyst and of a solvent forming an azeotrope with water, to form the reaction mixture, followed by
> ii) refluxing the said reaction mixture, with simultaneous or successive and alternating esterification reactions, by reaction of acrylic acid with a hydroxyl of the said polyol, and etherification reactions, via Michael addition reaction of a hydroxyl of the said polyol to a formed acrylate group and gradual removal of the esterification water, with
> iii) continuation of the reaction until all of the OH functions have been consumed by Michael addition, and
> iv) neutralization of the said acidic catalyst for recovery of the said final product, by removal of the said solvent, without any other specific purification step required.

12. Crosslinkable composition, **characterized in that** it comprises at least one product as defined according to one of Claims 1 to 10 or obtained via a process as defined according to Claim 11.

13. Composition according to Claim 12, **characterized in that**, for Mn greater than 1000, preferably greater than 1500, it also comprises at least one reactive diluent selected from acrylic monomers, which are preferably multifunctional.

14. Composition according to Claim 12 or 13, **characterized in that** it is a radiation-crosslinkable composition, in particular under UV radiation in the presence of a photoinitiating system or via an electron beam (EB) and in the absence of a photoinitiating system and/or via a thermal radical initiating system, in particular via a peroxide initiating system (P-cure) and/or via Michael addition (M-cure) or via a mixed system, in particular by dual crosslinking (dual cure).

15. Composition according to one of Claims 12 to 14, **characterized in that** it is a pigmented or non-pigmented coating composition, preferably chosen from paint, varnish, ink, adhesive and gel coat or a composition for 3D (three-dimensional) articles with successive layers or a moulding composition or a sealing composition or a composite composition or a chemical sealing composition.

16. Use of a product as defined according to one of Claims 1 to 10 or obtained via a process as defined according to Claim 11, in crosslinkable compositions, in particular having a low degree of shrinkage.

17. Use according to Claim 16, **characterized in that** it applies to pigmented or non-pigmented coating compositions, in particular paints, varnishes, inks, adhesives and gel coats or compositions for 3D (three-dimensional) articles with successive layers or moulding or sealing compositions or composite compositions or chemical sealing compositions.

18. Resulting finished products, **characterized in that** they result from the use of at least one product as defined according to one of Claims 1 to 10 or obtained via a process as defined according to Claim 11 or from the crosslinking of a composition as defined according to one of Claims 12 to 15 preferably chosen from pigmented or non-pigmented coatings, in particular chosen from paints, varnishes, inks, adhesives and gel coats or 3D (three-dimensional) articles with successive layers or moulded pieces or seals or composites or chemical seals.

**EP 2 999 690 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- JP 2010024380 A